# FASCICULE DE BREVET EUROPEEN

(11) **EP 1 017 418 B1**
(45) Date de publication et mention de la délivrance du brevet: **14.05.2008**
(21) Numéro de dépôt: 98913848.2
(22) Date de dépôt: 06.03.1998
(51) Int. Cl.: A61K 39/29, C07K 14/18

(54) **COMPOSITION VACCINALE DESTINEE A LA PREVENTION OU AU TRAITEMENT DES HEPATITES C**
IMPFSTOFF ZUR VORBEUGUNG BZW. BEHANDLUNG DER HEPATITIS C
VACCINE COMPOSITION FOR PREVENTING OR TREATING C HEPATITIS

(30) Priorité: 06.03.1997 FR 9702887
(43) Date de publication de la demande: 12.07.2000
(73) Titulaire: Sanofi Pasteur, 69367 Lyon Cedex 07 (FR)
(72) Inventeur: BARBAN, Véronique, F-69007 Lyon (FR)
(74) Mandataire: Gros, Florent
(86) Numéro de dépôt international: PCT/FR1998/000448
(87) Numéro de publication internationale: WO 1998/039030

(56) Documents cités:
- EP-A- 0 484 787
- LIU Q ET AL: "Regulated processing of hepatitis C virus core protein is linked to subcellular localization." JOURNAL OF VIROLOGY 71 (1). 1997. 657-662, XP002047845 cité dans la demande
- HUSSY P ET AL: "Hepatitis C virus core protein: Carboxy-terminal boundaries of two processed species suggest cleavage by a signal peptide peptidase." VIROLOGY 224 (1). 1996. 93-104, XP002070763
- BUKH J. ET AL.: "Sequence analysis of the core gene of 14 hepatitis C virus genotypes" PROCEEDINGS OF THE NATIONAL ACADEMY OF SCIENCES OF USA., vol. 91, no. 17, 16 août 1994, WASHINGTON US, pages 8239-8243, XP002047846 cité dans la demande

## Description

L'invention concerne une composition pharmaceutique destinée au traitement ou à la prophylaxie des infections induites par le virus de l'hépatite C (HCV).

Le virus de l'hépatite C (HCV) est l'agent responsable de la majeure partie des hépatites infectieuses de type non A non B. La séroprévalence des infections à VHC varie entre 0,3 et 1,5% dans la population mondiale, pouvant atteindre jusqu'à 18% dans certains pays en voie de développement. Des centaines de millions de personnes seraient ainsi infectées dans le monde. Neuf types et trente sous types de VHC ont été décrits. Les sous types peuvent être associés à une distribution géographique définie, le type 1b étant le plus répandu à travers le monde. L'évolution vers la forme chronique survient dans 50% des cas, environ 5 ans après la primo-infection. L'Hépatite Chronique Persistante qui est asymptomatique mais qui présente un titre élevé de virus circulant, est d'abord observée, puis l'Hépatite Chronique Active s'installe. Vingt pour cent des hépatites chroniques progressent en cirrhose du foie en une dizaine d'années. L'hépatocarcinome peut se développer dans les foies cirrhotiques.

Le virus de l'hépatite C (HCV) est un virus à ARN simple-brin positif. Sur la base de ressemblances structurales, le HCV a été rapproché des familles des flavivirus et des pestivirus.

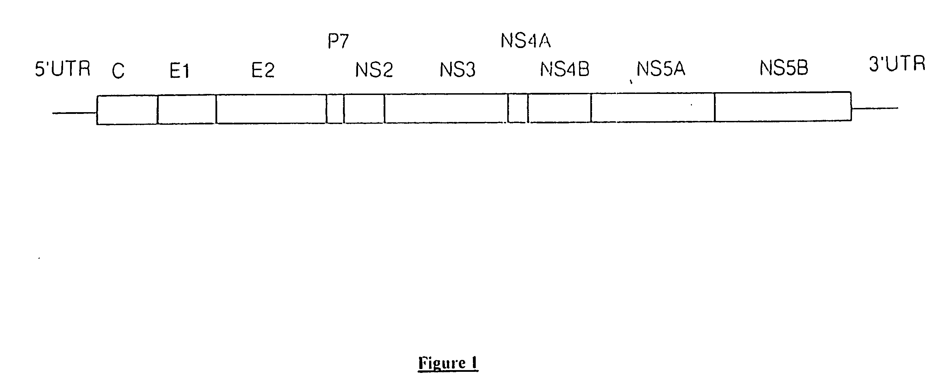

Lors d'un évènement infectieux, le génome du HCV est d'abord traduit en une polyprotéine précurseur d'environ 3000 acides aminés. Cette polyprotéine subit ensuite des clivages post-traductionnels pour donner différents précurseurs et protéines virales matures. Les protéines structurales du HCV sont localisées dans la région N-terminale de la polyprotéine. Tel que montré dans la figure 1, il s'agit plus particulièrement de la protéine de capside ou core (C ), des protéines d'enveloppe E1 et E2, qui sont présentes dans l'ordre suivant : NH2-C-E1-E2. Cette partie est clivée par les protéases de la cellule hôte (Grakoui A et al (1993)- J. Virol. 67 : 1385-1395).

La numérotation des acides aminés de la polyprotéine ainsi que de ses dérivés, qui est adoptée ci-après, est celle couramment utilisée et notamment présentée par Choo et al [PNAS (vol.88 : p.2451(1991)]. Ainsi, la protéine C correspond aux acides aminés en positions 1 à 191 de la polyprotéine et la protéine E1, aux acides aminés en positions 192 à 380. Dans la suite du texte, on convient de numéroter les acides aminés de la séquence de la protéine E1, de la position 192 à la position 380, 381, 382 ou 383. Dans la suite du texte, par souci de simplicité, on se réfère uniquement à la position C-terminale 380.

La protéine C issue du clivage direct de la polyprotéine comporte 191 acides aminés. Cette protéine C, aussi dénommée C191, peut elle-même être tronquée vers son extrémité C-terminale par clivage enzymatique pour donner une protéine de 173 acides aminés, dénommée C 173. Dans la suite du texte, le terme « protéine ou polypeptide C » désignera de préférence la forme C191.

La protéine C est un bon candidat vaccinal dans la mesure où c'est bien sûr une protéine de structure du virus et dans la mesure où la région codant pour cette protéine est relativement bien conservée par les différentes souches du HCV. On sait qu'une région de la protéine C capable de générer une forte réponse anticorps correspond aux 120 premiers acides aminés ; les 48 premiers acides aminés constituant le domaine antigénique majeur. Cependant, un obstacle majeur à son utilisation en tant que vaccin réside en ce que cette protéine est capable de transactiver des gènes appartenant à la cellule hôte, en particulier des gènes tels que des oncogènes, ce qui pourrait avoir entre autre pour conséquence d'induire un événement de carcinogénèse.

En effet, on a en particulier montré que la forme C173 était capable de translocation dans le noyau de la cellule hôte et de transactivation. La région de la protéine C responsable de la translocation dans le noyau et de l'activité régulatrice semble être localisée dans la partie N-terminale (123 premiers acides aminés).

Ainsi la région de la protéine C qui présente un intérêt d'un point de vue vaccinal est d'autre part responsable d'un effet toxique à l'égard de la cellule-hôte.

Liu, Q. et al (J. virol. (1997) 71 : 657-662) a montré qu'en utilisant une protéine C191 dont le site de clivage est muté en 173-174, on empêchait la migration de la protéine C173 dans le noyau par formation d'hétéromultimères entre C173 et C191. Malgré tout, comme on le verra ci après, la protéine C 191 mutée conserve une activité régulatrice même si c'est à un degré moindre.

De manière surprenante, on a maintenant mis en évidence qu'il était possible d'abolir l'activité régulatrice de la protéine C en la modifiant et en l'associant dans certaines conditions avec la protéine E1. La présente invention fournit des moyens pour abolir l'activité régulatrice en empêchant la migration de la protéine C dans le noyau. Cette migration n'a plus lieu en présence de la protéine E1 qui possède entre autre la propriété de s'ancrer dans le cytoplasme, au niveau du réticulum endoplasmique, et qui de manière inattendue, à la capacité d'y retenir la protéine C lorsque certaines conditions sont réalisées. La migration peut être abolie en réalisant par exemple une fusion des deux protéines, clivable ou non ; dans le cas ou elle est clivable, les produits générés doivent être capables d'interagir entre eux pour qu'il n'y ait pas fuite de l'un d'entre eux dans le noyau ; le complexe formé par le produit de clivage étant capable de s'ancrer dans le cytoplasme. L'équivalent d'une fusion peptidique clivable est un mélange en quantité équimolaire des éléments constituant la fusion.

C'est pourquoi l'invention a pour objet une composition pharmaceutique comprenant :
(i) Un polypeptide qui est incapable d'être clivé par une protéase dans une cellule de mammifère et qui comporte :
   (a) une première région correspondant au moins à la partie du polypeptide C du virus HCV, responsable de l'activité régulatrice dudit polypeptide C à l'encontre d'un ou plusieurs gènes, qui lorsqu'il comprend la région correspondant aux acides aminés 172 à 175 du polypeptide C du HCV comporte une mutation rendant inopérant le site de clivage 173/174 du polypeptide C du HCV ; et
   (b) une deuxième région correspondant au moins à une partie du polypeptide E1 dudit virus responsable de l'ancrage cytoplasmique du polypeptide E1; ou
(ii) Un polypeptide qui est susceptible d'être clivé par une protéase dans une cellule de mammifère et qui comporte :
   (a) une première région correspondant au moins à la partie du polypeptide C du virus HCV, responsable de l'activité régulatrice dudit polypeptide C à l'encontre d'un ou plusieurs gènes, et à la partie dudit polypeptide C responsable de l'interaction dudit polypeptide C avec la protéine E1 dudit virus, qui lorsqu'il comprend la région correspondant aux acides aminés 172 à 175 du polypeptide C du HCV comporte une mutation rendant inopérant le site de clivage 173/174 du polypeptide C du HCV ; et
   (b) une deuxième région correspondant au moins à une partie du polypeptide E1 dudit virus responsable de l'interaction du polypeptide E1 avec le polypeptide C dudit virus, et à une partie responsable de l'ancrage cytoplasmique du polypeptide E1 ; ou
(iii) Un mélange en quantité équimolaire comprenant :
   (a) un premier polypeptide qui comprend une première région correspondant au moins à la partie du polypeptide C du virus HCV responsable de l'activité régulatrice du dudit polypeptide C à l'encontre d'un ou plusieurs gènes et une deuxième région correspondant au moins à la partie dudit polypeptide C responsable de l'interaction dudit polypeptide C avec la protéine E1 dudit virus, ledit premier polypeptide comprenant la région correspondant aux acides aminés 172 à 175 du polypeptide C du HCV et comportant une mutation rendant inopérant le site de clivage 173/174 du polypeptide C du HCV; et
   (b) un deuxième polypeptide qui comprend une première région correspondant au moins à une partie du polypeptide E1 dudit virus responsable de l'interaction du polypeptide E1 avec le polypeptide C dudit virus et une deuxième région correspondant au moins à une partie du polypeptide E1 dudit virus responsable de l'ancrage cytoplasmique du polypeptide E1 ; ou -
(iv) une molécule d'ADN comprenant une séquence codant pour le polypeptide tel que décrit en (i), ou en (ii) de la présente revendication placée sous le contrôle nécessaire à son expression dans une cellule de mammifère ; et un support ou un diluent acceptable d'un point de vue pharmaceutique.

Sous un autre aspect de l'invention, l'invention a aussi pour objet une méthode de traitement ou de prévention d'une infection induite par le HCV selon laquelle on administre une composition pharmaceutique selon l'invention, à un mammifère, de préférence un humain, ayant besoin d'un tel traitement.

Par "polypeptide", on entend toute chaîne d'acides aminés liés entre eux de manière covalente, quelle que soit la longueur de la chaîne et quelles que soient les modifications post traductionnelles qui peuvent avoir lieu comme par exemple une lipidation. On peut également utiliser le terme protéine, de manière interchangeable.

Par "polypeptide C du HCV", on entend notamment un polypeptide C qui possède la séquence d'acides aminés telle que divulguée par Choo et al ainsi que tout autre polypeptide C provenant de toute autre souche et dont la séquence diffèrerait de celle de Choo et al. Par exemple, il peut s'agir des polypeptides C décrits dans Takeuchi K et al [Nucleic Acids Research 18 : 4626 (1990)], Houghton M et al.[Hepatology 14 : 381 (1991)], Delisse et al. [J. Hepatology.13,suppl. 4 (1991)], Bukh J et al [PNAS 91 : 8239 (1994)] et Hiroaki O et al [Intervirology 37 : 68 (1994)].

Par "polypeptide E1 du HCV", on entend notamment un polypeptide E1 qui possède la séquence d'acides aminés telle que divulguée par Choo et al ainsi que tout autre polypeptide E1 provenant de toute autre souche et dont la séquence diffèrerait de celle de Choo et al. Par exemple, il peut s'agir des polypeptides E1 décrits dans Hiroaki O et al [Intervirology 37 : 68 (1994), Grakoui et al. [J. Virol. 67 : 1385 (1993)], et Spaete et al. [Virology 188: 819 (1992)], Matsumia et al. [J. Virol. 66 : 1425], ou dans Kohara et al. [J. Gen. Virol. 73 : 2313 (1992)].

Ainsi, la séquence d'acides aminés du polypeptide C et celle du polypeptide E1 du HCV peut varier en fonction de la souche virale ; reflétant le phénomène de variance allélique. Par exemple, un virus est usuellement représenté par un ensemble de souches qui diffèrent entre elles par des caractéristiques alléliques mineures. Un polypeptide qui remplit la même fonction biologique dans différentes souches peut avoir une séquence d'acides aminés qui n'est pas la même pour toutes les souches. Une telle variation allélique se retrouve également au niveau de l'ADN.

Au niveau de la séquence d'acides aminés, les différences alléliques peuvent être constituées par une ou plusieurs substitutions, délétions ou additions d'acides aminés, qui n'altèrent pas la fonction biologique.

En ce qui concerne le polypeptide compris dans la composition pharmaceutique selon l'invention, on se doit d'envisager deux cas : soit le polypeptide est incapable d'être clivé par une protéase dans une cellule de mammifère, soit il est susceptible d'un tel clivage.

Par "partie du polypeptide C du HCV, responsable de l'activité régulatrice dudit polypeptide C à l'encontre d'un ou plusieurs gènes", on entend notamment toute partie du polypeptide C du HCV capable d'activer, de transactiver ou de supprimer la transcription ou l'expression d'un gène quelconque, selon un mécanisme quelconque. Ce gène peut être un gène eucaryote, un gène viral, un oncogène, ou un proto-oncogène.

Une partie du polypeptide C du HCV responsable de l'activité régulatrice dudit polypeptide peut notamment correspondre aux acides aminés en positions 38 à 43, 58 à 64, 66 à 71, 6 à 23, 39 à 74, 99 à 102, 101 à 121, 101 à 122, 58 à 121, 1 à 120. 1 à 121, 1 à 122, 1 à 123, 1 à 173 De manière préférée, une partie du polypeptide C du HCV responsable de l'activité régulatrice peut être une partie du polypeptide C allant de l'acide aminé en position 1 à l'acide aminé dans l'une des positions 48 à 191. A cette fin, une des positions 48 à 191 peut être par exemple, la position 119, 120, 121, 123 et 173.

Une partie du polypeptide C du HCV responsable de l'interaction dudit polypeptide C avec la protéine E1 du HCV peut notamment correspondre aux acides aminés en positions 151 à 173 ou en positions 173 à 191 du polypeptide C du HCV.

Une partie du polypeptide E1 du HCV responsable de l'ancrage cytoplasmique du polypeptide E1 peut être un domaine hydrophobe du polypeptide E1. De tels domaines hydrophobes sont par exemple localisés en positions 262 à 291, 370 à 380 et 330 à 380 du polypeptide E1.

Une partie du polypeptide E1 du HCV responsable de l'interaction du polypeptide C avec la protéine E1 peut être notamment le domaine C-terminal du polypeptide E1. de préférence le domaine en positions 330 à 380 ou en positions 370 à 380.

Dans un polypeptide utile aux fins de la présente invention, la première région peut être localisée du côté N- ou C-terminal du polypeptide, avantageusement du côté N-terminal ; de même la deuxième région peut être localisée du côté C- ou N-terminal, avantageusement du côté C-terminal. Selon un mode préféré, l'extrémité C-terminale de la première région peut être fusionnée par liaison peptidique à l'extrémité N-terminale de la deuxième région.

Lorsque le polypeptide compris dans la composition pharmaceutique selon l'invention comprend la région correspondant au moins aux acides aminés en positions 172 à 175 du polypeptide C du HCV, ce polypeptide comporte avantageusement une mutation rendant inopérant le site de clivage en position 173/174. Selon un mode préféré, une telle mutation est une mutation ponctuelle, réalisée dans l'une des positions 172 à 175. Elle peut être obtenue par exemple par délétion, addition ou substitution d'un ou plusieurs acides aminés, notamment par délétion, addition ou substitution d'un ou plusieurs acides aminés en position 172 à 175. De préférence, la mutation sera réalisée par substitution d'un ou deux acides aminés ; une double mutation par substitution étant tout particulièrement préférée. Selon un exemple particulier, le résidu existant naturellement en position 173 (sérine) pourra être notamment substitué par le résidu méthionine et le résidu existant naturellement en position 174 (phénylalanine) pourra être notamment substitué par le résidu leucine. D'une manière générale, il est à la portée de l'homme du métier de réaliser une ou des mutations capable de rendre inopérant le site de clivage en position 173/174 du polypeptide C du HCV.

Lorsque le polypeptide compris dans la composition pharmaceutique selon l'invention comprend la région correspondant au moins aux acides aminés en positions 190 à 193 de la polyprotéine du HCV, ce polypeptide comporte avantageusement une mutation rendant inopérant le site de clivage en position 191/192 de la polyprotéine du HCV. Selon un mode préféré, une telle mutation est une mutation ponctuelle réalisée dans l'une des positions 190 à 193. Elle peut être obtenue par exemple par délétion, addition ou substitution d'un ou plusieurs acides aminés, notamment par délétion, addition ou substitution d'un ou plusieurs acides aminés en positions 190 à 193. De préférence, la mutation sera réalisée par substitution d'un ou deux acides aminés ; une double mutation par substitution étant tout particulièrement préférée. Selon un exemple particulier, le résidu existant naturellement en position 191 (alanine) pourra notamment être substitué par le résidu valine et le résidu existant naturellement en position 192 (tyrosine) pourra notamment être substitué par le résidu asparagine. D'une manière générale, il est à la portée de l'homme du métier de réaliser une ou des mutations capable de rendre inopérant le site de clivage en position 191/192.

Lorsque le polypeptide utile aux fins de la présente invention comprend à la fois la région correspondant au moins aux acides aminés 190 à 193 de la polyprotéine du HCV et la région correspondant au moins aux acides aminés 172 à 175 du polypeptide C du HCV, le site de clivage 173/174 est rendu inopérant, de préférence les deux sites de clivage seront rendus inopérants. Lorsque seul le site 173/174 est rendu inopérant, le polypeptide est susceptible d'être clivé et dans ce cas-là, il est nécessaire que ce polypeptide possède une première région qui corresponde entre autre à la partie du polypeptide C responsable de l'interaction dudit polypeptide avec le polypeptide E1 et une deuxième région qui corresponde entre autre à la partie du polypeptide E1 responsable de l'interaction dudit polypeptide avec le polypeptide C.

Lorsqu'un polypeptide utile aux fins de la présente invention, est incapable d'être clivé par une protéase, il peut comporter un site de clivage à condition toutefois que ce site de clivage ne soit pas fonctionnel. Par exemple, dans le cas particulier d'un polypeptide constitué par le polypeptide C191 fusionné au polypeptide E1 et comportant une mutation rendant inopérant le site de clivage en position 191/192, le site de clivage en position 173/174 peut ne pas être muté ; toutefois, il ne sera pas fonctionnel ou peu, dans la mesure où le clivage en position 191/192 n'est plus possible. En effet on sait que le clivage en position 191/192 doit être réalisé pour que puisse avoir lieu le clivage en position 173/174.

Selon un mode particulier, un polypeptide utile aux fins de la présente invention est incapable d'être clivé par une protéase et comporte :
(a) une première région qui correspond substantiellement au domaine du polypeptide C allant de l'acide aminé en position 1 à l'acide aminé dans l'une des positions 120 à 173 ; et
(b) une deuxième région qui correspond substantiellement à une domaine du polypeptide E1 comportant un moins une région hydrophobe, par exemple au domaine du polypeptide E1 allant de l'acide aminé en position 192 à l'acide aminé en position 380, ou de l'acide aminé en position 330 à l'acide aminé en position 380, ou de l'acide aminé en position 260 à l'acide aminé en position 290, ou de l'acide aminé en position 260 à l'acide aminé en position 380.

Selon un mode particulier, un polypeptide utile aux fins de la présente invention est incapable d'être clivé par une protéase et comporte :
(a) une première région qui correspond substantiellement au domaine du polypeptide C allant de l'acide aminé en position 1 à l'acide aminé dans l'une des positions 120 à 191, qui lorsqu'il comprend la région correspondant au moins aux acides aminés en position 172 à 175 du polypeptide du HCV comporte une mutation rendant inopérant le site de clivage en position 173/174 ; et
(b) une deuxième région qui correspond substantiellement à un domaine du polypeptide E1 comportant au moins une région hydrophobe, par exemple au domaine du polypeptide E1 allant de l'acide aminé en position 192 à l'acide aminé en position 380, ou de l'acide aminé en position 330 à l'acide aminé en position 380, ou de l'acide aminé en position 260 à l'acide aminé en position 290, ou de l'acide aminé en position 260 à l'acide aminé en position 380 ;
à condition que ledit polypeptide ne comporte pas de site de clivage 191/192 ou bien lorsque le site de clivage est reconstitué, alors une mutation est introduite pour le rendre inopérant.

De manière avantageuse, la première région du polypeptide utile aux fins de la présente invention correspond aux acides aminés en positions 1 à 191 du polypeptide C du HCV et/ou la deuxième région de ce polypeptide correspond au moins aux acides aminés en positions 192 à 380 du polypeptide E1 du HCV. De manière particulièrement préférée, la première et la deuxième région sont comme définies ci-avant dans ce même paragraphe, l'acide aminé en position 191 étant fusionné par liaison peptidique à l'acide aminé en position 192. Selon un mode particulier, le polypeptide est constitué par la première et la deuxième région telles que définies ci-avant dans ce même paragraphe.

Lorsque le polypeptide utile aux fins de la présente invention est tel que décrit au paragraphe précédemment, il comporte impérativement une mutation rendant inopérant le site de clivage en position 173/174. De préférence, les deux sites de clivage en position 173/174 et en position 191/192 sont rendus inopérants.

Un mélange « en quantité equimolaire » utile aux fins de la présente invention comprend avantageusement :
(a) un premier polypeptide comportant une région qui correspond au moins à la partie du polypeptide C du virus HCV responsable de l'activité régulatrice dudit polypeptide C à l'encontre d'un ou plusieurs gènes, ledit premier polypeptide comprenant la région correspondant au moins aux acides aminés en position 172 à 175 du polypeptide C du HCV et comportant une mutation rendant inopérant le site de clivage en position 173/174, et à la partie dudit polypeptide C responsable de l'interaction dudit polypeptide C avec le polypeptide E1 dudit virus ; et
(b) un deuxième polypeptide comportant une région correspondant à une partie du polypeptides E1 dudit virus responsable de l'interaction du polypeptide E1 avec le polypeptide C dudit virus et à une partie du polypeptide E1 dudit virus responsable de l'ancrage cytoplasmique du polypeptide E1.

Les parties des polypeptides C et E1 responsables des propriétés nommées aux points (a) et (b) du paragraphe précédent peuvent être telles que décrites ci-avant pour le polypeptide de fusion.

De manière préféré, le premier polypeptide du mélange comporte et de manière tout particulièrement préférée est constitué par, une région correspondant aux acides aminés en positions 1 à 191 du polypeptide C (C191) du HCV. Dans ce dernier cas, le site de clivage en position 173/174 doit être rendu inopérant par mutation. Cette mutation peut être mise en oeuvre comme cela est décrit ci-avant pour le polypeptide de fusion.

De manière préférée, le deuxième polypeptide du mélange comporte et de manière tout particulièrement préférée est constitué par, une région correspondant aux acides aminés en positions 192 à 380 du polypeptide E1 du HCV.

Aux fins de la présente invention, une molécule d'ADN peut être un simple fragment d'ADN linéaire, ou bien un plasmide ou bien encore un vecteur viral tel qu'un vecteur pox.

Un polypeptide, un mélange ou une molécule d'ADN tels que décrits dans la présente demande, sont d'un interêt tout particulier lorsqu'ils sont utilisés pour la fabrication d'un médicament destiné au traitement ou à la prévention des infections induites par le HCV. Ils sont notamment utiles en immunothérapie des infections induites par le HCV, tout particulièrement une molécule d'ADN.

Enfin l'invention concerne une méthode d'induction d'une réponse immunitaire à l'encontre du HCV chez un mammifère, selon laquelle on administre audit mammifère une quantité efficace d'un point de vue immunologique d'une composition selon l'invention afin de développer une réponse immunitaire. L'invention concerne également une méthode de prévention ou de traitement d'une infection induite par le HCV, selon laquelle on administre à un individu une quantité prophylactiquement ou thérapeutiquement efficace d'une composition selon l'invention.

Les méthodes et les compositions pharmaceutiques selon l'invention peuvent traiter ou prévenir des infections à ECV et par conséquent, les maladies hépatiques associées à de telles infections. Il s'agit en particulier, des hépatites chroniques persistantes; des hépatites chroniques actives; des cirrhoses du foie et des hépatocarcinomes.

Une composition selon l'invention peut être administrée par n'importe quelle voie conventionnelle en usage dans le domaine des vaccins, en particulier par voie parentérale (*e*.*g*. sous-cutanée, intradermique, intramusculaire, intraveineuse, ou intrapéritonéale). Le choix de la voie d'administration dépend d'un certain nombre paramètres tel que la nature du principe actif, polypeptide ou molécule d'ADN, l'adjuvant associé au polypeptide ou à la molécule d'ADN.

Une composition selon l'invention peut comprendre outre un polypeptide ou un mélange de polypeptide utiles aux tins de la présente invention, au moins un autre antigène du HCV tel que la protéine E2 ou encore tel qu'une protéine non structurale NS1, NS2. NS3, NS4 ou NS5, ou une sous-unité, fragment, homologue, mutant ou dérivé de ces antigènes.

Un polypeptide, un mélange ou une molécule d'ADN utiles aux fins de la présente invention peut être formulé dans ou avec des liposomes, de préférence des liposomes neutres ou anioniques, des microspères, des ISCOMs ou des pseudo-particules virales (VLPs), afin de favoriser le ciblage de la protéine ou du polypeptide ou d'augmenter la réponse immunitaire. L'homme du métier dispose de ces composés sans difficulté : par exemple voir Liposomes : A Practical Approach, RRC New ED, IRL press (1990).

Des adjuvants autres que les liposomes peuvent être aussi utilisés. Un grand nombre sont connus de l'homme du métier. De tels adjuvants sont référencés ci-après :

Pour administration parentérale, on cite notamment des composés d'aluminium tels que l'hydroxyde d'aluminium, le phosphate d'aluminium et l'hydroxyphosphate d'aluminium. L'antigène peut être adsorbé ou précipité sur un composé d'aluminium selon des méthodes standards. D'autres adjuvants utiles pour administration parentérale, incluent notamment le polyphosphazène (WO 95/2415), le DC-chol (3 bétâ-[N-(N',N'-dimethyl aminométhane)-carbamoyl] cholestérol) (USP 5 283 185 et WO 96/14831), le QS-21 (WO 88/9336) et le RIBI d'ImmunoChem (Hamilton, MT).

L'administration peut avoir lieu en dose unique ou répétée une ou plusieurs fois après un certain délai. Le dosage approprié varie en fonction de divers paramètres, par exemple, de l'individu traité (adulte ou enfant) de l'antigène vaccinal lui-même du mode et de la fréquence d'administration, de la présence ou absence d'adjuvant et si présent, du type d'adjuvant et de l'effet désiré (*e*.*g*. protection ou traitement), comme cela peut être déterminé par l'homme de l'art.

Une composition selon l'invention peut être fabriquée de manière conventionnelle. En particulier on associe un polypeptide, un mélange ou une molécule d'ADNcompris dans la composition selon l'invention avec un diluant ou un support acceptable d'un point de vue pharmaceutique *e*.*g*. de l'eau ou une solution saline tel qu'un tampon de sel de phosphate (PBS). En général, le diluant ou le support est sélectionné sur la base du mode et de la voie d'administration et de pratiques pharmaceutiques standards. Des diluants et des supports acceptables d'un point de vue pharmaceutique ainsi que tout ce qui est nécessaire à leur usage dans des formulations pharmaceutiques sont décrits dans Remington's Pharmaceutical Sciences, un texte de référence standard dans ce domaine et dans le USP/NP.

L'invention est illustrée ci-après, par références aux figures suivantes.
La figure 1 est une représentation schématique du génome du HCV qui est constitué d'ARN avec ses régions 5' et 3' non traduites indiquées par des lignes, et la phase ouverte de lecture de la polyprotéine précurseuse indiquée sous la forme d'un rectangle.
La figure 2 représente les inserts dérivés du génome du HCV qui sont testés dans des plasmides pRC. Les séquences dérivées du génome du HCV sont représentées par un rectangle et les résidus mutés sont indiqués par des points.

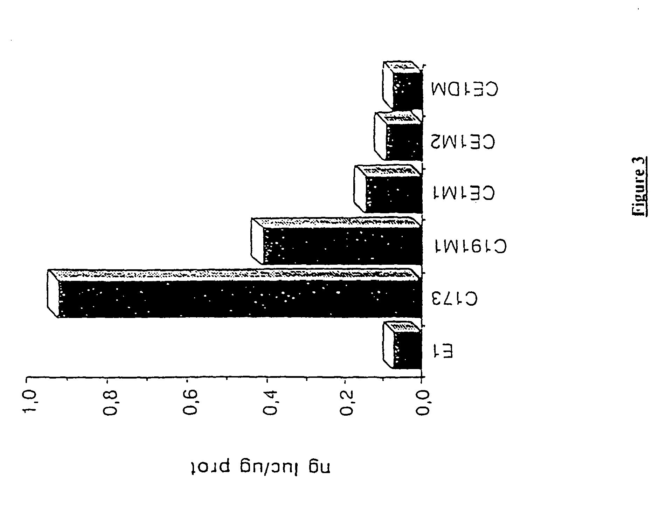

La figure 3 est un diagramme représentant l'activité luciférase mesurée pour chacune des constructions dont certaines sont mutées au niveau d'un ou de plusieurs site(s) de clivage. L'identité de l'insert testé apparait en abscisse tandis que la quantité de luciferase produite par rapport à la quantité totale de protéines produites apparait en ordonnées.

### EXEMPLE 1 : Construction de plasmides recombinants et mutagénèse dirigée

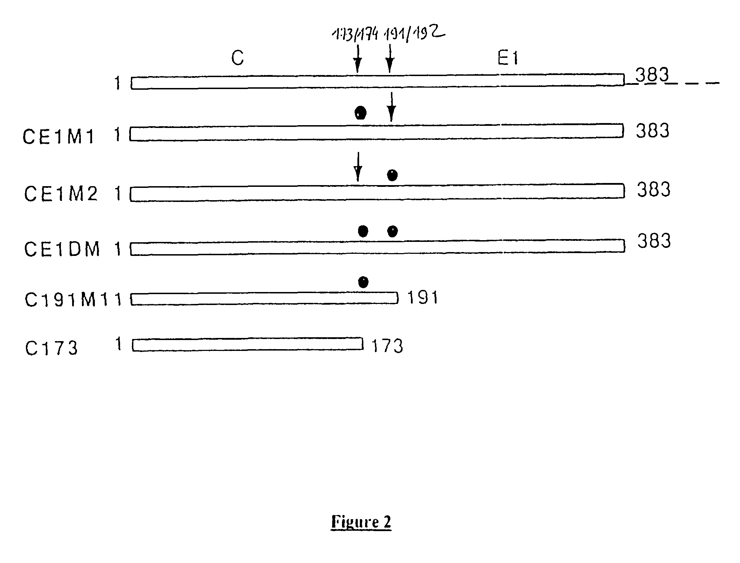

Les constructions dénommées pRC/E1, pRC/CE1M1, pRC/CE1M2 et pRC/CE1M1M2(aussi appellé pRC/CE1DM), pRC/C191M1, et pRC/C173, qui sont utilisées ci-après dans l'exemple 2, ont été décrites dans : Liu Q et al. J. Virol. 71 : 657 (1997). Les inserts utilisés sont représentées à la figure 2. Toutes les constructions sont réalisées dans le vecteur pRC qui provient de InVitrogen (ref: V780-20). Le vecteur pRC porte le gène de l'ampicilline et permet l'expression des inserts sous le contrôle d'un promoteur CMV. Des mutations dénommées M1 et M2 sont présentes dans les constructions pRC/C 191M1, pRC/CE1M1, RC/CE1M2 et pRC/CE1M1M2. Elles ont été générés par mutagénèse dirigée réalisée par PCR. La mutation dénommée M1 correspond au remplacement des acides aminés Serine¹⁷³ et Phe¹⁷⁴ de la protéine C par les acides aminés methionine et leucine, respectivement. La mutation dénommée M2 correspond au remplacement des acides aminés alanine¹⁹¹ et tyrosine¹⁹² de la protéine CE1 par les acides aminés valine et asparagine, respectivement.

Les plasmides exprimant les gènes rapporteurs de la luciferase et de la β-galactosidase ont été construits par modification du vecteur pUC 18 (Appligene; ref : 161131). L'expression des gènes est sous contrôle du promoteur immediate early 1 (ie1) du CMV humain. Des sequences issues de la région 3' du gène bovin de l'hormone de croissance, ont par ailleurs été rajoutées en 3' des gènes pour stabiliser les ARNm. Ces plasmides portent de plus un gène de résistance à l'ampicilline.

### EXEMPLE 2 : Transfection de cellules par les plasmides

Des cellules CHO-K1 (ATCC CCL 61) sont conservées dans du milieu α-MEM [Nature 230:310 (1971)], supplémenté par 10 % de Sérum de Veau Foetal (SVF) (Hyclone, ref : A 1115-L) et 20 % de Diméthyl Sulfoxyde (DMSO) dans l'azote liquide. Ces cellules sont cultivées sous atmosphère humide à 37 °C avec 5 % de CO₂ et 95 % d'air. Pour effectuer des sous cultures, le milieu éliminé et le tapis cellulaire est rinçé avec ml de tampon phosphate (PBS). Le surnageant est ensuite éliminé avant ajout de 1.5 ml de trypsine par flasques de 75 cm² (trypsine à 0,025 %). Après une incubation de 10 mn à l'étuve à 37 °C, la réaction est arretée par ajout de 10 ml de milieu α-MEM contenant 10 % SVF. Les cellules sont comptées sur cellule de Malassez après une dilution au demi en bleu Trypan à 0.02 %, 5.10⁵ cellules sont ensuite ensemencées dans des boites de 6 cm de diamètre avec du milieu complet.

Les cellules CHO sont ensuite cotransfectées avec un des plasmides recombinants (pHCV) décrits ci-dessus et un plasmide rapporteur (pCMV) qui contient soit le gène de la β-galactosidase sous le contrôle du promoteur CMV (pCMV β-gal), soit le gène de la luciferase sous le contrôle du promoteur CMV (pCMV Luc).

Pour cela. 5 µg d'ADN (4,5 µg de plasmide pHCV/ 0,5 µg de plasmide pCMV) sont dilués dans 500 µl de milieu OPTI-MEM (Gibco), et mélangés avec 14 µl de lipofectamine diluée dans 500 µl du même milieu. Les deux solutions sont mélangées et incubées 20 min à température ambiante pour permettre la formation des complexes ADN-liposomes.

Le mésange ADN-liposome dilué avec 2 ml d'OPTI-MEM est ensuite rajouté sur les cellules après élimination du milieu de culture et rinçage en PBS. Après une incubation de 5 heures, le milieu est de nouveau change et 48 h après la transfection, il est alors possible de tester l'expression transitoire des gènes recombinants.

### EXEMPLE 3 : Mise en évidence de l'activité régulatrice des constructions sur des gènes rapporteurs

Les cellules transfectées sont lysées à l'aide du réactif "Luciferase Cell Culture Lysis Ragent" (Promega, Luciferase Assay System). 100 µl de substrat sont ajoutés à 100 µl de surnageant cellulaire, directement par l'injecteur du bioluminomètré (Lumat LB/9501/16 de Berthold) qui mesure la quantité de lumière émise (Relative Light Units) pendant 10 secondes. La quantité de lumière émise est ensuite convertie en nanogrammes de protéines par ml de lysat cellulaire, par comparaison avec une courbe standard établie à l'aide de luciferase purifiée.

Les résultats qui sont présentés à la figure 2 montrent qu'une mutation ponctuelle à l'acide aminé 191 dans la construction CE1M2 abolit l'effet transactivateur. Une mutation ponctuelle à l'acide aminé 173 (dans la construction CE1M1 abolit l'effet transactivateur uniquement dans le cas ou C est fusionné à E1. Une double mutation aux acides aminés 173 et 191 abolit l'effet transactivateur.

## Revendications

1. Une composition pharmaceutique comprenant:
(i) Un polypeptide qui est incapable d'être clivé par une protéase dans une cellule de mammifère et qui comporte :
(a) une première région correspondant au moins à la partie du polypeptide C du virus HCV, responsable de l'activité régulatrice dudit polypeptide C à l'encontre d'un ou plusieurs gènes, qui lorsqu'il comprend la région correspondant aux acides aminés 172 à 175 du polypeptide C du HCV comporte une mutation rendant inopérant le site de clivage 173/174 du polypeptide C du HCV ; et
(b) une deuxième région correspondant au moins à une partie du polypeptide E1 dudit virus responsable de l'ancrage cytoplasmique du polypeptide E1; ou
(ii) Un polypeptide qui est susceptible d'être clivé par une protéase dans une cellule de mammifère et qui comporte :
(a) une première région correspondant au moins à la partie du polypeptide C du virus HCV, responsable de l'activité régulatrice dudit polypeptide C à l'encontre d'un ou plusieurs gènes, et à la partie dudit polypeptide C responsable de l'interaction dudit polypeptide C avec la protéine E1 dudit virus, qui lorsqu'il comprend la région correspondant aux acides aminés 172 à 175 du polypeptide C du HCV comporte une mutation rendant inopérant le site de clivage 173/174 du polypeptide C du HCV ; et
(b) une deuxième région correspondant au moins à une partie du polypeptide E1 dudit virus responsable de l'interaction du polypeptide E1 avec le polypeptide C dudit virus, et à une partie responsable de l'ancrage cytoplasmique du polypeptide E1; ou
(iii) Un mélange en quantité équimolaire comprenant :
(a) un premier polypeptide qui comprend une première région correspondant au moins à la partie du polypeptide C du virus HCV responsable de l'activité régulatrice du dudit polypeptide C à l'encontre d'un ou plusieurs gènes et une deuxième région correspondant au moins à la partie dudit polypeptide C responsable du l'interaction dudit polypeptide C avec la protéine E1 dudit virus, ledit premier polypeptide comprenant la région correspondant aux acides aminés 172 à 175 du polypeptide C du HCV et comportant une mutation rendant inopérant le site de clivage 173/174 du polypeptide C du HCV ; et
(b) un deuxième polypeptide qui comprend une première région correspondant an moins à une partie du polypeptide E1 dudit virus responsable de l'interaction du polypeptide E1 avec le polypeptide C dudit virus et une deuxième région correspondant au moins à une partie du polypeptide E1 dudit virus responsable de l'ancrage cytoplasmique du polypeptide E1 ; ou
(iv) une molécule d'ADN comprenant une séquence codant pour le polypeptide tel que décrit en (i), ou en (ii) de la présente revendication placée sous le contrôle nécessaire à son expression dans une cellule de mammifère ; et
un support ou un diluent acceptable d'un point de vue pharmaceutique.

2. Une composition pharmaceutique selon la revendication 1 comprenant :
(i) Un polypeptide tel que décrit en (i) de la revendication 1 qui comporte :
(a) une première région correspondant au moins aux acides aminés en positions 1 à 123 du polypeptide C du HCV ; et
(b) une deuxième région correspondant au moins aux acides aminés en positions 260 à 290 ou en positions 330 à 380 du polypeptide E1 du HCV ; ou
(ii) Un polypeptide tel que décrit en (ii) de la revendication 1 qui comporte :
(a) une première région correspondant au moins aux acides aminés en positions 1 à 123 du polypeptide C du HCV et aux acides aminés en positions 173 à 191 dudit polypeptide ; et
(b) une deuxième région correspondant au moins aux acides aminés en positions 330 à 380 du polypeptide E1 du HCV et, de manière optionnelle, aux acides aminés en positions 260 à 290 dudit polypeptide ; ou
(iii) Un mélange tel que décrit en (iii) de la revendication 1 dans lequel :
(a) La première région du premier polypeptide correspond au moins aux acides aminés en positions 1 à 123 du polypeptide C du HCV et la deuxième région correspond au moins aux acides aminés en positions 173 à 191 du polypeptide C du HCV ; et
(b) La première et la deuxième région du deuxième polypeptide correspondent au moins aux acides aminés en positions 330 à 380 du polypeptide E1 du HCV et, de manière optionnelle, aux acides aminés en positions 260 à 290 dudit polypeptide; ou
(iv) Une molécule d'ADN comprenant une séquence codant pour le polypeptide tel que décrit en (i) ou en (ii) de la présente revendication, placée sous le contrôle des éléments nécessaires à son expression dans une cellule de mammifère ; et
Un support ou un diluent acceptable d'un point de vue pharmaceutique.

3. Une composition selon la revendication 1 ou 2, comprenant :
(i) Un polypeptide tel que décrit en (i) ou (ii) selon la revendication 1 ou 2, dans lequel la première région est localisée du côté N-terminal du polypeptide et la deuxième région est localisée du côté C-terminal du polypeptide ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

4. Une composition selon la revendication 3, comprenant :
(i) Un polypeptide tel que décrit en (i) de la revendication 3, dans lequel l'extrémité C- terminale de la première région est fusionnée par liaison peptidique à l'extrémité N-terminale de la deuxième région ; ou
(ii) Une molécule d'ADN comprenant une séquence codant pour le polypeptide tel que décrit en (i) de la présente revendication placée sous le contrôle des éléments nécessaires à son expression dans une cellule de mammifère.

5. Une composition selon l'une des revendications 1 à 4, comprenant :
(i) Un polypeptide tel que décrit en (i) de la revendication 1, selon laquelle la première région comprend la région correspondant aux acides aminés 172 à 175 du polypeptide C du HCV et au moins une mutation dans l'une des positions 172 à 175 ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

6. Une composition selon la revendication 5, comprenant :
(i) Un polypeptide tel que décrit en (i) de la revendication 5, dans lequel la au moins une mutation est obtenue par substitution ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

7. Une composition selon la revendication 6, comprenant :
(i) Un polypeptide tel que défini en (i) de la revendication 6, dans lequel le résidu serine existant naturellement en position 173 est substitué par le résidu methionine et le résidu phenylalanine existant naturellement en position 174 est substitué par le résidu leucine ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

8. Une composition selon l'une des revendications 1 à 7, comprenant :
(i) Un polypeptide tel que décrit en (i) dans l'une des revendications 1 à 7 ou en (ii) dans la revendication 1 ou 2, qui lorsqu'il comprend la région correspondant aux acides aminés 190 à 193 de la polyprotéine du HCV comporte une mutation rendant inopérant le site de clivage 191/192 de la polyprotéine du HCV ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

9. Une composition selon la revendication 8, comprenant :
(i) Un polypeptide tel que décrit en (i) de la revendication 8, comprenant au moins une mutation dans l'une des positions 190 à 193 ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

10. Une composition selon la revendication 9, comprenant :
(i) Un polypeptide tel que décrit en (i) de la revendication 9, dans lequel la au moins une mutation est obtenue par substitution ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

11. Une composition selon la revendication 10, comprenant :
(i) Un polypeptide tel que défini en (i) de la revendication 10, dans lequel le résidu alanine existant naturellement en position 191 est substitué par le résidu valine et le résidu tyrosine existant naturellement en position 192 est substitué par le résidu asparagine ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

12. Une composition selon l'une des revendications 1 à 11 comprenant :
(i) Un polypeptide tel que décrit en (i) de l'une des revendications 1 à 11 ou en (ii) dans la revendication 1 ou 2, comportant une première région correspondant aux acides aminés en positions 1 à 191 du polypeptide C du HCV ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

13. Une composition selon l'une des revendications 1 à 12 comprenant :
(i) Un polypeptide tel que décrit en (i) de l'une des revendications 1 à 12 ou en (ii) de la revendication 1 ou 2, comportant une deuxième région correspondant au moins aux acides aminés en positions 192 à 380 du polypeptide E1 du HCV ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

14. Une composition selon les revendications 12 et 13.

15. Une composition selon la revendication 14, comprenant :
(i) Un polypeptide tel que décrit en (i) des revendications 12 et 13, qui comporte une première région correspondant aux acides aminés en positions 1 à 191 du polypeptide C du HCV et une deuxième région correspondant aux acides aminés en positions 192 à 380 du polypeptide E1 du HCV, et dans lequel l'acide aminé en position 191 est fusionné par liaison peptidique à l'acide aminé en position 192 ; ou
(ii) Une molécule d'ADN comprenant une séquence codant pour le polypeptide tel que décrit en (i) de la présente revendication, placée sous le contrôle des éléments nécessaires à son expression dans une cellule de mammifère.

16. Une composition selon les revendications 5, 6, ou 7 et 15.

17. Une composition selon les revendications 8, 9, 10 ou 11 et 15.

18. Une composition selon les revendications 16 et 17.

19. Une composition comprenant un mélange tel que décrit en (iii) de la revendication 1 ou 2, dans lequel le premier polypeptide du mélange comporte au moins une substitution dans l'une des positions 172 à 175.

20. Une composition selon la revendication 19, dans lequel le premier polypeptide du mélange est muté par substitution du résidu sérine existant naturellement en position 173 par le résidu méthionine et par substitution du résidu phenylalanine existant naturellement en position 174 par le résidu leucine.

21. Une composition selon l'une des revendications 1, 2, 19 et 20, dans laquelle le premier polypeptide tel que défini en (iii) de la revendication 1 ou 2, et tel que défini dans la revendication 19 ou 20, comporte une région correspondant aux acides aminés en position 1 à 191 du polypeptide C du HCV.

22. Une composition selon l'une des revendications 1, 2, 19, 20 et 21, dans laquelle le deuxième polypeptide tel que défini en (iii) de la revendication 1 ou 2, comprend une région correspondant aux acides aminés en positions 192 à 380 du polypeptide E1 du HCV.

23. Utilisation d'une composition selon l'une des revendications 1 à 22 dans la fabrication d'un médicament pour prévenir ou traiter les infections induites par le virus HCV.

## Claims

1. Pharmaceutical composition comprising:
(i) A polypeptide which is incapable of being cleaved by a protease in a mammalian cell and which contains:
(a) a first region corresponding at least to the portion of the C polypeptide of the HCV virus responsible for the regulatory activity of said C polypeptide toward one or more genes, which when it comprises the region corresponding to the amino acids 172 to 175 of the C polypeptide of HCV, contains a mutation making inoperative the cleavage site 173/174 of the C polypeptide of HCV; and
(b) a second region corresponding at least to a portion of the E1 polypeptide of said virus responsible for the cytoplasmic anchorage of the E1 polypeptide; or
(ii) A polypeptide which is capable of being cleaved by a protease in a mammalian cell and which contains:
(a) a first region corresponding at least to the portion of the C polypeptide of the HCV virus responsible for the regulatory activity of said C polypeptide toward one or more genes and to the portion of said C polypeptide responsible for the interaction of said C polypeptide with the E1 protein of said virus, which when it comprises the region corresponding to the amino acids 172 to 175 of the C polypeptide of HCV, contains a mutation making inoperative the cleavage site 173/174 of the C polypeptide of HCV; and
(b) a second region corresponding at least to a portion of the E1 polypeptide of said virus responsible for the interaction of the E1 polypeptide with the C polypeptide of said virus and to a portion responsible for the cytoplasmic anchorage of the E1 polypeptide; or
(iii) A mixture in equimolar quantity comprising:
(a) a first polypeptide which comprises a first region corresponding at least to the portion of the C polypeptide of the HCV virus responsible for the regulatory activity of said C polypeptide toward one or more genes and a second region corresponding at least to the portion of said C polypeptide responsible for the interaction of said C polypeptide with the E1 protein of said virus, said first polypeptide comprising the region corresponding to the amino acids 172 to 175 of the C polypeptide of HCV and containing a mutation making inoperative the cleavage site 173/174 of the C polypeptide of HCV; and
(b) a second polypeptide which comprises a first region corresponding at least to a portion of the E1 polypeptide of said virus responsible for the interaction of the E1 polypeptide with the C polypeptide of said virus and a second region corresponding at least to a portion of the E1 polypeptide of said virus responsible for the cytoplasmic anchorage of the E1 polypeptide; or
(iv) a DNA molecule comprising a sequence encoding the polypeptide as described in (i) or in (ii) of the present claim, placed under the control necessary for its expression in a mammalian cell; and
a pharmaceutically acceptable carrier or diluent.

2. Pharmaceutical composition according to Claim 1, comprising:
(i) a polypeptide as described in (i) of Claim 1, which contains:
(a) a first region corresponding at least to the amino acids at positions 1 to 123 of the C polypeptide of HCV; and
(b) a second region corresponding at least to the amino acids at positions 260 to 290 or at positions 330 to 380 of the E1 polypeptide of HCV; or
(ii) a polypeptide as described in (ii) of Claim 1, which contains:
(a) a first region corresponding at least to the amino acids at positions 1 to 123 of the C polypeptide of HCV and to the amino acids at positions 173 to 191 of said polypeptide; and
(b) a second region corresponding at least to the amino acids at positions 330 to 380 of the E1 polypeptide of HCV and, optionally, to the amino acids at positions 260 to 290 of said polypeptide; or
(iii) a mixture as described in (iii) of Claim 1, in which:
(a) the first region of the first polypeptide corresponds at least to the amino acids at positions 1 to 123 of the C polypeptide of HCV and the second region corresponds at least to the amino acids at positions 173 to 191 of the C polypeptide of HCV; and
(b) the first and the second regions of the second polypeptide correspond at least to the amino acids at positions 330 to 380 of the E1 polypeptide of HCV and, optionally, to the amino acids at positions 260 to 290 of said polypeptide; or
(iv) a DNA molecule comprising a sequence encoding the polypeptide as described in (i) or in (ii) of the present claim, placed under the control of the elements necessary for its expression in a mammalian cell; and
a pharmaceutically acceptable carrier or diluent.

3. Composition according to Claim 1 or 2, comprising:
(i) a polypeptide as described in (i) or (ii) according to Claim 1 or 2, in which the first region is located on the N-terminal side of the polypeptide and the second region is located on the C-terminal side of the polypeptide; or
(ii) a DNA molecule comprising a sequence encoding the polypeptide as described in (i) of the present claim, placed under the control of the elements necessary for its expression in a mammalian cell.

4. Composition according to Claim 3, comprising:
(i) a polypeptide as described in (i) of Claim 3, in which the C-terminal end of the first region is fused by peptide bonding to the N-terminal end of the second region; or
(ii) a DNA molecule comprising a sequence encoding the polypeptide as described in (i) of the present claim, placed under the control of the elements necessary for its expression in a mammalian cell.

5. Composition according to one of Claims 1 to 4, comprising:
(i) a polypeptide described in (i) of Claim 1, according to which the first region comprises the region corresponding to the amino acids 172 to 175 of the C polypeptide of HCV and at least one mutation at one of positions 172 to 175; or
(ii) a DNA molecule comprising a sequence encoding the polypeptide as described in (i) of the present claim, placed under the control of the elements necessary for its expression in a mammalian cell.

6. Composition according to Claim 5, comprising:
(i) a polypeptide as described in (i) of Claim 5, in which the at least one mutation is obtained by substitution; or
(ii) a DNA molecule comprising a sequence encoding the polypeptide as described in (i) of the present claim, placed under the control of elements necessary for its expression in a mammalian cell.

7. Composition according to Claim 6, comprising:
(i) a polypeptide as defined in (i) of Claim 6, in which the serine residue naturally existing at position 173 is substituted by the methionine residue and the phenylalanine residue naturally existing at position 174 is substituted by the leucine residue; or
(ii) a DNA molecule comprising a sequence encoding the polypeptide as described in (i) of the present claim, placed under the control of the elements necessary for its expression in a mammalian cell.

8. Composition according to one of Claims 1 to 7, comprising:
(i) a polypeptide as described in (i) in one of Claims 1 to 7 or in (ii) in Claim 1 or 2, which when it comprises the region corresponding to the amino acids 190 to 193 of the HCV polyprotein, contains a mutation making inoperative the cleavage site 191/192 of the polyprotein of HCV; or
(ii) a DNA molecule comprising a sequence encoding the polypeptide as described in (i) of the present claim, placed under the control of the elements necessary for its expression in a mammalian cell.

9. Composition according to Claim 8, comprising:
(i) a polypeptide as described in (i) of Claim 8, comprising at least one mutation in one of positions 190 to 193; or
(ii) a DNA molecule comprising a sequence encoding the polypeptide as described in (i) of the present claim, placed under the control of the elements necessary for its expression in a mammalian cell.

10. Composition according to Claim 9, comprising:
(i) a polypeptide as described in (i) of Claim 9, in which the at least one mutation is obtained by substitution; or
(ii) a DNA molecule comprising a sequence encoding the polypeptide as described in (i) of the present claim, placed under the control of the elements necessary for its expression in a mammalian cell.

11. Composition according to Claim 10, comprising:
(i) a polypeptide as defined in (i) of Claim 10, in which the alanine residue naturally existing at position 191 is substituted by the valine residue and the tyrosine residue naturally existing at position 192 is substituted by the asparagine residue; or
(ii) a DNA molecule comprising a sequence encoding the polypeptide as described in (i) of the present claim, placed under the control of the elements necessary for its expression in a mammalian cell.

12. Composition according to one of Claims 1 to 11, comprising:
(i) a polypeptide as described in (i) of one of Claims 1 to 11 or in (ii) in Claim 1 or 2, containing a first region corresponding to the amino acids at positions 1 to 191 of the C polypeptide of HCV; or
(ii) a DNA molecule comprising a sequence encoding the polypeptide as described in (i) of the present claim, placed under the control of the elements necessary for its expression in a mammalian cell.

13. Composition according to one of Claims 1 to 12, comprising:
(i) a polypeptide as described in (i) of one of Claims 1 to 12 or in (ii) of Claim 1 or 2, containing a second region corresponding at least to the amino acids at positions 192 to 380 of the E1 polypeptide of HCV; or
(ii) a DNA molecule comprising a sequence encoding the polypeptide as described in (i) of the present claim, placed under the control of the elements necessary for its expression in a mammalian cell.

14. Composition according to Claims 12 and 13.

15. Composition according to Claim 14, comprising:
(i) a polypeptide as described in (i) of Claims 12 and 13, which contains a first region corresponding to the amino acids at positions 1 to 191 of the C polypeptide of HCV and a second region corresponding to the amino acids at positions 192 to 380 of the E1 polypeptide of HCV, and in which the amino acid at position 191 is fused by peptide bonding to the amino acid at position 192; or
(ii) a DNA molecule comprising a sequence encoding the polypeptide as described in (i) of the present claim, placed under the control of the elements necessary for its expression in a mammalian cell.

16. Composition according to Claims 5, 6 or 7 and 15.

17. Composition according to Claims 8, 9, 10 or 11 and 15.

18. Composition according to Claims 16 and 17.

19. Composition comprising a mixture as defined in (iii) of Claim 1 or 2, in which the first polypeptide of the mixture contains at least one substitution at one of positions 172 to 175.

20. Composition according to Claim 19, in which the first polypeptide of the mixture is mutated by substitution of the serine residue naturally existing at position 173 with the methionine residue and by substitution of the phenylalanine residue naturally existing at position 174 by the leucine residue.

21. Composition according to one of Claims 1, 2, 19 and 20, in which the first polypeptide as defined in (iii) of Claim 1 or 2, and as defined in Claim 19 or 20, contains a region corresponding to the amino acids at positions 1 to 191 of the C polypeptide of HCV.

22. Composition according to one of Claims 1, 2, 19, 20 and 21, in which the second polypeptide as defined in (iii) of Claim 1 or 2, comprises a region corresponding to the amino acids at positions 192 to 380 of the E1 polypeptide of HCV.

23. Use of a composition according to one of Claims 1 to 22 in the manufacture of a medicament for preventing or treating infections induced by the HCV virus.

## Patentansprüche

1. Pharmazeutische Zusammensetzung, umfassend:
(i) ein Polypeptid, das nicht in der Lage ist, durch eine Protease in einer Säuger-Zelle gespalten zu werden, und das folgendes einschließt:
(a) eine erste Region, die mindestens dem Teil des Polypeptids C des Virus-HCV entspricht, der für die regulatorische Aktivität des Polypeptids C gegenüber einem oder mehreren Genen verantwortlich ist, die, wenn sie die Region, die den Aminosäuren 172 bis 175 des Polypeptids C des HCV entspricht, einschließt, eine Mutation einschließt, die die Spaltstelle 173/174 des Polypeptids C des HCV inoperabel macht; und
(b) eine zweite Region, die mindestens einem Teil des Polypeptids E1 des Virus entspricht, die für die cytoplasmatische Verankerung des Polypeptids E1 verantwortlich ist; oder
(ii) ein Polypeptid, das einer Spaltung durch eine Protease in einer Säuger-Zelle zugänglich ist und das folgendes einschließt:
(a) eine erste Region, die mindestens dem Teil des Polypeptids C des HCV Virus entspricht, der für die regulatorische Aktivität des Polypeptids C gegenüber einem oder mehreren Genen verantwortlich ist, und dem Teil des Polypeptids C, der für die Wechselwirkung des Polypeptids C mit dem E1-Protein des Virus verantwortlich ist, der, wenn er die Region, die den Aminosäuren 172 bis 175 des Polypeptids C des HCV entspricht, einschließt, eine Mutation einschließt, die die Spaltstelle 173/174 des Polypeptids C des HCV inoperabel macht; und
(b) eine zweite Region, die mindestens einem Teil des Polypeptids E1 des Virus, der für die Wechselwirkung des Polypeptids E1 mit dem Polypeptid C des Virus verantwortlich ist, und einem Teil, der für die cytoplasmatische Verankerung des Polypeptids E1 verantwortlich ist, entspricht; oder
(iii) ein Gemisch in äquimolarer Menge, das folgendes einschließt:
(a) ein erstes Polypeptid, das eine erste Region, die mindestens dem Teil des Polypeptids C des HCV-Virus entspricht, der für die regulatorische Aktivität des Polypeptids C gegenüber einem oder mehreren Genen verantwortlich ist, und eine zweite Region, die mindestens dem Teil des Polypeptids C entspricht, der für die Wechselwirkung des Polypeptids C mit dem Protein E1 des Virus verantwortlich ist, einschließt, wobei das erste Polypeptid die Region, die den Aminosäuren 172 bis 175 des Polypeptids C des HCV entspricht, und eine Mutation, die die Spaltstelle 173/174 des Polypeptids C des HCV inoperabel macht, einschließt; und
(b) ein zweites Polypeptid, das eine erste Region, die mindestens einem Teil des Polypeptids E1 des Virus entspricht, der für die Wechselwirkung des Polypeptids E1 mit dem Polypeptid C des Virus verantwortlich ist, und eine zweite Region, die mindestens einem Teil des Polypeptids E1 des Virus entspricht, der für die cytoplasmatische Verankerung des Polypeptids E1 verantwortlich ist, einschließt; oder
(iv) ein DNA-Molekül, das eine Sequenz einschließt, die das Polypeptid, wie unter (i) oder unter (ii) des vorliegenden Anspruchs beschrieben, codiert, die unter die für seine Expression in einer Säuger-Zelle notwendige Kontrolle gestellt ist; und
einen Träger oder ein Verdünnungsmittel, das pharmazeutisch verträglich ist.

2. Pharmazeutische Zusammensetzung nach Anspruch 1, die folgendes einschließt:
(i) Ein Polypeptid, wie unter (i) des Anspruchs 1 beschrieben, das folgendes einschließt:
(a) eine erste Region, die mindestens den Aminosäuren in den Positionen 1 bis 123 des Polypeptids C des HCV entspricht; und
(b) eine zweite Region, die mindestens den Aminosäuren in den Positionen 260 bis 290 oder in den Positionen 330 bis 380 des Polypeptids E1 des HCV entspricht; oder
(ii) ein Polypeptid, wie in (ii) des Anspruchs 1 beschrieben, das folgendes einschließt:
(a) eine erste Region, die mindestens den Aminosäuren in den Positionen 1 bis 123 des Polypeptids C des HCV und den Aminosäuren in den Positionen 173 bis 191 des Polypeptids entspricht; und
(b) eine zweite Region, die mindestens den Aminosäuren in den Positionen 330 bis 380 des Polypeptids E1 des HCV und, gegebenenfalls, den Aminosäuren in den Positionen 260 bis 290 des Polypeptids entspricht; oder
(iii) ein Gemisch wie in (iii) des Anspruchs 1 beschrieben, wobei:
(a) die erste Region des ersten Polypeptids mindestens den Aminosäuren in den Positionen 1 bis 123 des Polypeptids C des HCV entspricht und die zweite Region mindestens den Aminosäuren in den Positionen 173 bis 191 des Polypeptids C des HCV entspricht; und
(b) die erste und die zweite Region des zweiten Polypeptids mindestens den Aminosäuren in den Positionen 330 bis 380 des Polypeptids E1 des HCV und, gegebenenfalls, den Aminosäuren in den Positionen 260 bis 290 des Polypeptids entspricht; oder
(iv) ein DNA-Molekül, das eine Sequenz einschließt, die das Polypeptid, wie unter (i) oder unter (ii) des vorliegenden Anspruches beschrieben, codiert, die unter die Kontrolle der für seine Expression in einer Säuger-Zelle notwendigen Elemente gestellt ist; und
einen pharmazeutisch verträglichen Träger oder ein pharmazeutisch verträgliches Verdünnungsmittel.

3. Zusammensetzung nach Anspruch 1 oder 2, die folgendes einschließt:
(i) ein Polypeptid, wie unter (i) oder unter (ii) nach Anspruch 1 oder 2 beschrieben, wobei die erste Region auf der N-terminalen Seite des Polypeptids lokalisiert ist und die zweite Region auf der C-terminalen Seite des Polypeptids lokalisiert ist; oder
(ii) ein DNA-Molekül, das eine Sequenz einschließt, die das Polypeptid, wie unter (i) des vorliegenden Anspruches beschrieben, codiert, die unter die Kontrolle der für seine Expression in einer Säuger-Zelle notwendigen Elemente gestellt ist.

4. Zusammensetzung nach Anspruch 3, die folgendes einschließt:
(i) ein Polypeptid, wie unter (i) des Anspruchs 3 beschrieben, wobei das C-terminale Ende der ersten Region über eine peptidische Bindung mit dem N-terminalen Ende der zweiten Region fusioniert ist; oder
(ii) ein DNA-Molekül, das eine Sequenz einschließt, die das Polypeptid, wie unter (i) des vorliegenden Anspruches beschrieben, codiert, die unter die Kontrolle der für seine Expression in einer Säuger-Zelle notwendigen Elemente gestellt ist.

5. Zusammensetzung nach einem der Ansprüche 1 bis 4, die folgendes einschließt:
(i) ein Polypeptid, wie unter (i) des Anspruchs 1 beschrieben, nach dem die erste Region die Region, die den Aminosäuren 172 bis 175 des Polypeptids C des HCV entspricht, und mindestens eine Mutation in einer der Positionen 172 bis 175 einschließt; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

6. Zusammensetzung nach Anspruch 5, die folgendes einschließt:
(i) ein Polypeptid, wie unter (i) des Anspruchs 5 beschrieben, wobei die mindestens eine Mutation durch Substitution erhalten wird; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

7. Zusammensetzung nach Anspruch 6, die folgendes einschließt:
(i) ein Polypeptid, wie unter (i) des Anspruchs 6 definiert, wobei der Serin-Rest, der von Natur aus in Position 173 vorkommt, durch den Methionin-Rest ersetzt ist und der Phenylalanin-Rest, der von Natur aus in Position 174 vorkommt, durch den Leucin-Rest substituiert ist; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

8. Zusammensetzung nach einem der Ansprüche 1 bis 7, die folgendes einschließt:
(i) ein Polypeptid, wie unter (i) in einem der Ansprüche 1 bis 7 oder unter (ii) in dem Anspruch 1 oder 2 beschrieben, das, wenn es die Region einschließt, die den Aminosäuren 190 bis 193 des Polyproteins des HCV entspricht, eine Mutation einschließt, die die Spaltstelle 191/192 des Polyproteins des HCV inoperabel macht; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

9. Zusammensetzung nach Anspruch 8, die folgendes einschließt:
(i) ein Polypeptid, wie unter (i) des Anspruchs 8 beschrieben, das mindestens eine Mutation in einer der Positionen 190 bis 193 einschließt; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

10. Zusammensetzung nach Anspruch 9, die folgendes einschließt:
(i) ein Polypeptid, wie unter (i) des Anspruchs 9 beschrieben, wobei die mindestens eine Mutation durch Substitution erhalten wird; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

11. Zusammensetzung nach Anspruch 10, die folgendes einschließt:
(i) ein Polypeptid, wie unter (i) des Anspruchs 10 definiert, wobei der Alanin-Rest, der von Natur aus in Position 191 vorkommt, durch den Valin-Rest ersetzt ist und der Tyrosin-Rest, der von Natur aus in Position 192 vorkommt, durch den Asparagin-Rest ersetzt ist; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

12. Zusammensetzung nach einem der Ansprüche 1 bis 11, die folgendes einschließt:
(i) ein Polypeptid, wie unter (i) in einem der Ansprüche 1 bis 11 oder unter (ii) in dem Anspruch 1 oder 2 beschrieben, das eine erste Region einschließt, die den Aminosäuren in den Positionen 1 bis 191 des Polypeptids C des HCV entspricht; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

13. Zusammensetzung nach einem der Ansprüche 1 bis 12, die folgendes einschließt:
(i) ein Polypeptid, wie unter (i) in einem der Ansprüche 1 bis 12 oder unter (ii) in dem Anspruch 1 oder 2 beschrieben, das eine zweite Region einschließt, die mindestens den Aminosäuren in den Positionen 192 bis 380 des Polypeptids E1 des HCV entspricht; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

14. Zusammensetzung nach den Ansprüchen 12 und 13.

15. Zusammensetzung nach Anspruch 14, die folgendes einschließt:
(i) ein Polypeptid, wie unter (i) der Ansprüche 12 und 13 beschrieben, das eine erste Region einschließt, die den Aminosäuren in den Positionen 1 bis 191 des Polypeptids C des HCV entspricht, und eine zweite Region, die den Aminosäuren in den Positionen 192 bis 380 des Polypeptids E1 des HCV entspricht, und in dem die Aminosäure in Position 191 über peptidische Bindung der Aminosäure in Position 192 fusioniert ist; oder
(ii) ein DNA-Molekül, das eine Sequenz einschließt, die das Polypeptid, wie unter (i) des vorliegenden Anspruchs beschrieben, codiert, die unter die Kontrolle der für seine Expression in einer Säuger-Zelle notwendigen Elemente gestellt ist.

16. Zusammensetzung nach den Ansprüchen 5, 6 oder 7 und 15.

17. Zusammensetzung nach den Ansprüchen 8, 9, 10 oder 11 und 15.

18. Zusammensetzung nach den Ansprüchen 16 und 17.

19. Zusammensetzung, die ein Gemisch, wie unter (iii) des Anspruchs 1 oder 2 beschrieben, einschließt, wobei das erste Polypeptid des Gemisches mindestens eine Substitution in einer der Positionen 172 bis 175 einschließt.

20. Zusammensetzung nach Anspruch 19, wobei das erste Polypeptid des Gemisches durch Substitution des Sarin-Restes, der von Natur aus in Position 173 vorkommt, durch den Methionin-Rest und durch Substitution des Phenylalanin-Restes, der von Natur aus in Position 174 vorkommt, durch den Leucin-Rest mutiert ist.

21. Zusammensetzung nach einem der Ansprüche 1, 2, 19 und 20, wobei das erste Polypeptid, wie unter (iii) des Anspruchs 1 oder 2 definiert, und wie in Anspruch 19 oder 20 definiert, eine Region einschließt, die den Aminosäuren in Position 1 bis 191 des Polypeptids C des HCV entspricht.

22. Zusammensetzung nach einem der Ansprüche 1, 2, 19, 20 und 21, wobei das zweite Polypeptid, wie unter (iii) des Anspruchs 1 oder 2 definiert, eine Region einschließt, die den Aminosäuren in Position 192 bis 380 des Polypeptids E1 des HCV entspricht.

23. Verwendung einer Zusammensetzung nach einem der Ansprüche 1 bis 22 bei der Herstellung eines Medikaments zur Prävention oder zur Behandlung der durch das HCV-Virus ausgelösten Infektionen.
